## Europäisches Patentamt
## European Patent Office
## Office européen des brevets

(19)

(11) Veröffentlichungsnummer: **0 147 737**
**B1**

(12)
# EUROPÄISCHE PATENTSCHRIFT

(45) Veröffentlichungstag der Patentschrift:
**31.05.89**

(21) Anmeldenummer: **84115375.2**

(22) Anmeldetag: **13.12.84**

(51) Int. Cl.⁴: **A 61 B 8/14**

(54) Ultraschall-Tomographiegerät.

(30) Priorität: **27.12.83 DE 3347200**

(43) Veröffentlichungstag der Anmeldung:
**10.07.85 Patentblatt 85/28**

(45) Bekanntmachung des Hinweises auf die Patenterteilung:
**31.05.89 Patentblatt 89/22**

(84) Benannte Vertragsstaaten:
**AT DE FR GB NL**

(56) Entgegenhaltungen:
**EP-A-0 099 018
DE-A-3 224 464
FR-A-2 332 531
GB-A-2 023 830**

(73) Patentinhaber: **Siemens Aktiengesellschaft Berlin und München, Wittelsbacherplatz 2, D-8000 München 2 (DE)**

(72) Erfinder: **Breimesser, Fritz, Dompfaffstrasse 49, D-8520 Erlangen (DE)**
Erfinder: **Hassler, Dietrich, Flurweg 3, D-8525 Uttenreuth (DE)**
Erfinder: **Hundt, Eckart, Dr., Ferd- Kobell- Strasse 38, D-8013 Haar (DE)**
Erfinder: **Maderlechner, Gerd, Arnika- Strasse 5, D-8031 Gröbenzell (DE)**
Erfinder: **Trautenberg, Elmar, Dr., Steinacher Strasse 32a, D-8511 Fürth (DE)**

EP 0 147 737 B1

**Beschreibung**

Die Erfindung bezieht sich auf ein Ultraschall-Tomographiegerät mit einem Ultraschall-Sende-/Empfangssystem, das einen Ultraschallkopf enthält und das ein Untersuchungsobjekt aus unterschiedlichen Winkelbereichen zeilenweise abtastet. Das Ultraschall-Sende-/Empfangssystem ist mit einem Rotationsantrieb zur Rotation um eine Rotationsachse versehen.

Es ist ein Ultraschall-Tomographiegerät vorgeschlagen worden, das mit einem Ultraschall-Sende-/Empfangssystem zur zeilenweisen Abtastung eines Untersuchungsobjektes aus unterschiedlichen Winkelrichtungen vorgesehen ist. Das Ultraschall-Sende-/Empfangssystem umfaßt einzelne oder Gruppen von Schallköpfen, die das Untersuchungsobjekt aus unterschiedlichen Winkelrichtungen zeilenweise abtasten, die in koronaren oder koronar-ähnlichen Abtastebenen auf unterschiedliche Objekttiefen fokussierbar sind und denen eine Verstelleinrichtung zum Verstellen des Neigungswinkels der jeweiligen Sende-/Empfangskeule sowie eine Zeittorschaltung zum abschnittsweisen Erfassen von Signaldaten aus den unterschiedlichen Objekttiefen in den Fokusbereichen zugeordnet sind. Die Gruppen von Ultraschallköpfen können unterschiedlich ausgebildet sein. So können die Ultraschallköpfe Wandlerelemente umfassen, die beispielsweise als ein Linear-Array, als ein Array für elektronisch gesteuerten Sektor-Scan oder als ein Ring-Array ausgebildet sind. Der Ultraschallkopf kann aber auch einen einzelnen Ultraschallschwinger umfassen, der Bestandteil beispielsweise eines langsam rotierenden Sektorabtasters ist. Außerdem können die Ultraschallköpfe auch am Umfang eines Kreises versetzt angeordnet sein. Die Ringscheibe rotiert zusammen mit den mechanischen Sektorabtastern wieder um die durch das Untersuchungsobjekt gehende Achse. Zur Erzeugung der Sektorabtastfelder können die Sektorabtaster eine reine Schwenkbewegung ausführen. Außerdem können die einzelnen Sektorabtaster auch rundumrotierende Abtaster sein. Die Scheibenrotationsfrequenz ist in den beiden Fällen sehr viel niedriger als die Frequenz, mit der die Sektorabtaster geschwenkt oder gedreht werden. So beträgt beispielsweise die Drehgeschwindigkeit der Ringscheibe zusammen mit dem Sektorabtaster beispielsweise etwa 0,1 Hz. Die Schwenk- oder Drehfrequenz der Sektorabtaster liegt hingegen beispielsweise etwa bei 3 bis 4 Hz. Ferner kann als Ultraschallkopf auch ein Ultraschall-Array vorgesehen werden. Dieses Ultraschall-Array kann beispielsweise ein Linear-Array oder ein Array mit elektronisch gesteuertem Strahlschwenk für Sektorabtastung, das wieder entlang einem Kreisbogen langsam um die durch das Untersuchungsobjekt gehende Achse gedreht wird. Das Linear-Array ist vorzugsweise ein Mehrzeilen-Array, d. h., die einzelnen Wandlerelemente sind matrixartig angeordnet. Unterschiedliche Aperturen beim Senden und/oder Empfangen erhält man durch übliche elektronische Zu- oder wieder Wegschaltung von Einzelelementen zu unterschiedlich großen Strahlergruppen. Neben einem Linear-Array, das das Untersuchungsobjekt im Linear-Scan abtastet, ist auch noch ein Ring-Array vorgesehen, wobei die Strahlschwenkung und Strahlfortschaltung entlang dem Ring-Array rein elektronisch erfolgt. Siehe die nicht vorveröffentlichte unter Artikel 54(3) (EPÜ) fallende Europäische Patentanmeldung EP-A-0 099 018).

Der Erfindung liegt nun die Aufgabe zugrunde, ein Ultraschall-Tomographiegerät dieser Art zu verbessern, insbesondere sollen mehrere parallele Schnittebenen ohne zusätzliche mechanische Bewegung nacheinander oder sogar wenigstens annähernd gleichzeitig abgetastet werden.

Diese Aufgabe wird erfindungsgemäß gelöst mit den kennzeichnenden Merkmalen des Anspruchs 1. Der Ultraschallkopf ist ein Linear-Array, das aus mehreren linear angeordneten Wandlerelementen, vorzugsweise mehreren hundert Wandlerelementen, besteht. Dieses Array ist zu einer Schnittebene des Untersuchungsobjektes senkrecht angeordnet und ist zusätzlich um seine Längsachse drehbar. Mehrere Gruppen jeweils gemeinsam gesteuerter Wandlerelemente bilden jeweils eine Strahlapertur. Diese Strahlaperturen, die jeweils beispielsweise aus mehreren, vorzugsweise etwa 15, insbesondere aus etwa 30, linear angeordneten Wandlerelementen bestehen, werden gleichzeitig elektronisch fortgeschaltet, beispielsweise gemäß einer Gruppenfortschaltung, vorzugsweise gemäß einem Halbschrittverfahren und insbesondere gemäß einem modifizierten Halbschrittverfahren. Jeweils zwischen zwei benachbarten angesteuerten Gruppen ist wenigstens eine Gruppe vorgesehen, die abgeschaltet ist. Durch diese Ausgestaltung dieses Ultraschall-Tomographiegerätes erreicht man, daß mehrere parallele Schnittebenen wenigstens annähernd gleichzeitig abgetastet werden.

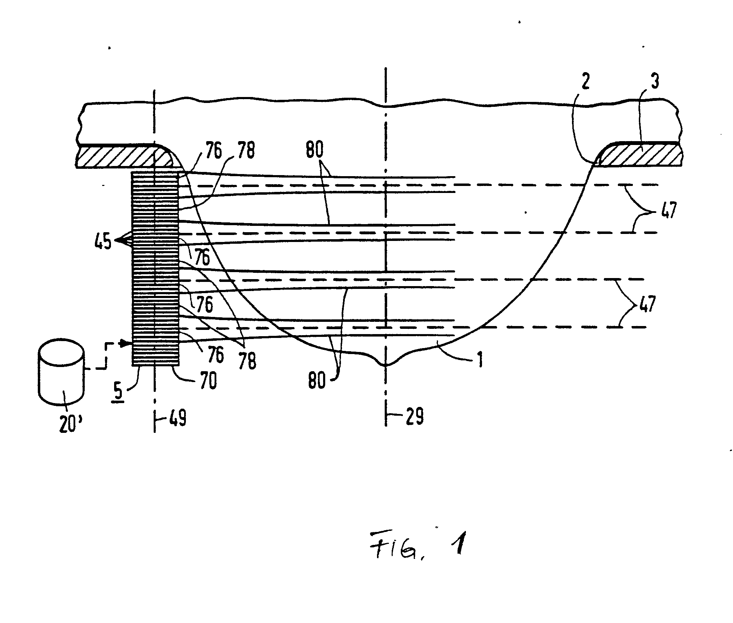

Zur weiteren Erläuterung wird auf die Zeichnung Bezug genommen, in der ein Ausführungsbeispiel des Ultraschall-Tomographiegerätes nach der Erfindung schematisch veranschaulicht ist.

In der dargestellten Ausführungsform eines Ultraschall-Tomographiegerätes ragt beispielsweise eine weibliche Brust als Untersuchungsobjekt 1 durch ein Applikationsloch 2 einer Platte 3, die Bestandteil eines Plattentisches ist, auf auf dem die Patientin liegt. Ein Ultraschall-Sende-/Empfangssystem 5 enthält einen Ultraschallkopf 70 und tastet aus unterschiedlichen Winkelbereichen das Untersuchungsobjekt 1 zeilenweise ab. Außerdem ist das Ultraschall-Sende/Empfangssystem 5 mit einem Rotationsantrieb 20' zur Drehung um eine Rotationsachse 29 versehen. Als Ultraschallkopf 70 ist ein Linear-Array aus mehreren Wandlerelementen 45 vorgesehen, das zu einer Schnittebene 47 des Untersuchungsobjektes 1 senkrecht angeordnet ist. Ferner ist das Linear-Array zusätzlich um seine Längsachse 49 drehbar angeordnet. Mehrere Gruppen 76, die jeweils beispielsweise aus mehreren, vorzugsweise 15, insbesondere aus etwa 30 linear angeordneten Wandlerelementen 45 bestehen, bilden jeweils eine Strahlapertur. Außerdem ist zwischen zwei benachbarten angesteuerten Gruppen 76 wenigstens eine Gruppe 78 vorgesehen, die abgeschaltet ist, damit die einzelnen Strahlungsdruckfelder 80 der angesteuerten Gruppen 76 sich nicht gegenseitig stören. Diese einzelnen angesteuerten Gruppen 76 sind jeweils mit einer elektronischen Fokussierung des Schaltfeldes und mit einem Parallel-Scan des Schallfeldes durch elektronische Fortschaltung versehen. Außerdem ist es auch möglich jeweils den Ultraschallstrahl der angesteuerten Gruppe 76 elektronisch zu schwenken.

Mit diesen Ausgestaltungen erhält man ein Ultraschall-Tomographiegerät, das einerseits ohne allzu großen technischen Aufwand brustwandnahe Koronarschnittbilder herstellt und andererseits wenigstens annähernd gleichzeitig mehrere parallele Schnittebenen 47 des Untersuchungsobjektes 1 abtastet.

Unter Umständen ist es ratsam ein Ring-Array vorzusehen, bei dem mehrere Gruppen 76 jeweils gemeinsam gesteuerter Wandlerelemente 45 jeweils eine Strahlapertur bilden. Auch bei dieser weiteren Ausgestaltung des Ultraschall-Tomographiegerätes ist zwischen zwei benachbarten angesteuerten Gruppen 76 wenigstens eine Gruppe 78 vorgesehen, die abgeschaltet ist. Durch diese Gestaltung erhält man ein Ultraschall-Tomographiegerät, das brustwandnahe Koronarschnittbilder oder wenigstens annähernd gleichzeitig weitere parallele Schnittebenen 47 des Untersuchungsobjektes 1 abtastet ohne dabei das Ultraschall-Sende-/Empfangssystem 5 mechanisch fortzubewegen.

**Patentansprüche**

1. Ultraschall-Tomographiegerät mit einem Ultraschall-Sende-/Empfangssystem (5), das einen Ultraschallkopf (70) enthält und das ein Untersuchungsobjekt (1) aus unterschiedlichen Winkelbereichen zeilenweise abtastet, und bei dem das Ultraschallsende-/Empfangssystem (5) mit einem Rotationsantrieb (20') zur Drehung um eine Rotationsachse (29) versehen ist, dadurch gekennzeichnet, daß als Ultraschallkopf (70) ein Linear-Array aus mehreren Wandlerelementen (45) vorgesehen ist, das zu einer Schnittebene (47) des Untersuchungobjektes (1) senkrecht und zusätzlich um seine zur Rotationsachse (29) parallele Längsachse (49) angeordnet ist, daß mehrere Gruppen (76) jeweils gemeinsam gesteuerter Wandlerelemente (45) jeweils eine Strahlapertur bilden und daß zwischen zwei benachbarten angesteuerten Gruppen (76) wenigstens eine Gruppe (78) vorgesehen ist, die abgeschaltet ist.

2. Ultraschall-Tomographiegerät nach Anspruch 1, dadurch gekennzeichnet, daß die einzelnen Gruppen (76, 78) jeweils mit einer elektronischen Fokussierung des Schallfeldes und mit einem Parallel-Scan des Schallfeldes durch elektronische Fortschaltung versehen sind.

**Claims**

1. Ultrasonic tomography apparatus having an ultrasonic transmitting/receiving system (5) which contains an ultrasonic head (70) and scans an examination object (1) from different angular ranges line by line and in which the ultrasonic transmitting/receiving system (5) is provided with a rotational drive (20') for the purposes of rotation about an axis of rotation (29), characterised in that a linear array of several transducer elements (45) is provided as the ultrasonic head (70), the array being arranged perpendicular to a sectional plane (47) of the examination object (1) and in addition rotatable about its longitudinal axis (49) which is parallel to the axis of rotation (29), in that several groups (76) of, in each case, jointly controlled transducer elements (45) each form a beam aperture and in that there is provided between two adjacent activated groups (76) at least one group (78) which is switched off.

2. Ultrasonic tomography apparatus according to claim 1, characterised in that the individual groups (76, 78) are provided, in each case, with electronic focussing of the sonic field and with a parallel scan of the sonic field through electronic continuous switching.

**Revendications**

1. Appareil de tomographie à ultrasons comportant un système d'émission/réception d'ultrasons (5), qui contient une tête ultrasonore (70) et explore par balayage ligne-par-ligne un objet à examiner (1) sous différentes plages angulaires et dans lequel le système d'émission/réception d'ultrasons (5) comporte un dispositif d'entraînement en rotation (20'), destiné à réaliser une rotation autour d'un axe de rotation (29), caractérisé par le fait qu'il est prévu, comme tête ultrasonore (70), un réseau linéaire formé de plusieurs éléments transducteurs (45) et disposé perpendiculairement à un plan de coupe (47) de l'objet à examiner (1), de manière à pouvoir, en outre, tourner autour de son axe longitudinal (49) parallèle à l'axe de rotation (29),

EP 0 147 737 B1

que plusieurs groupes (76) d'éléments transducteurs (45) commandés respectivement en commun forment chacun un angle d'ouverture du faisceau, et qu'entre deux groupes commandés voisins (76) il est prévu au moins un groupe (78), qui est débranché.

2. Appareil de tomographie à ultrasons suivant la revendication 1, caractérisé par le fait que les différents groupes (76, 78) sont pourvus respectivement d'un système de focalisation électronique du champ acoustique et d'un système de balayage parallèle du champ acoustique sous l'effet d'un système d'avance électronique.

4

FIG. 1